Europäisches Patentamt
European Patent Office
Office européen des brevets

(19)

(11) Veröffentlichungsnummer: **0 053 765**
**A1**

(12)

# EUROPÄISCHE PATENTANMELDUNG

(21) Anmeldenummer: 81109944.9

(22) Anmeldetag: 27.11.81

(51) Int. Cl.³: C 07 D 277/32

(30) Priorität: 10.12.80 DE 3046408

(43) Veröffentlichungstag der Anmeldung: 16.06.82 Patentblatt 82/24

(84) Benannte Vertragsstaaten: BE CH DE FR GB IT LI NL

(71) Anmelder: BAYER AG
Zentralbereich Patente, Marken und Lizenzen
D-5090 Leverkusen 1, Bayerwerk(DE)

(72) Erfinder: Beck, Gunther, Dr.
Am Mittelberg 19
D-5090 Leverkusen 1(DE)

(54) **Verfahren zur Herstellung von Trichlorthiazol.**

(57) Man erhält Trichlorthiazol der Formel

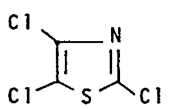

(I),

wenn man Chloralformamid der Formel

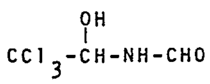

(II)

pro Mol mit mindestens zwei Mol Thionylchlorid unter Zusatz von 0,01 bis 10 Volumen-% eines N-alkylsubstituierten aliphatischen Säureamids, bezogen auf Thionylchlorid, und mit mindestens einem Mol Schwefel bei Temperaturen beginnend bei Raumtemperatur bis zu einem Bereich von 150-250°C umsetzt.

Trichlorthiazol kann als Zwischenprodukt zur Herstellung von bekannten herbiziden Wirkstoffen verwendet werden.

EP 0 053 765 A1

BAYER AKTIENGESELLSCHAFT 5090 Leverkusen, Bayerwerk

Zentralbereich
Patente, Marken und Lizenzen Bi-by-c
IVa/ZP

## Verfahren zur Herstellung von Trichlorthiazol

Die Erfindung betrifft ein neues Verfahren zur Herstellung von Trichlorthiazol.

Es sind zwei Verfahren bekannt, die, ausgehend von Chloralformamid, in zwei Stufen zum Trichlorthiazol führen.

Beim ersten Verfahren wird aus dem Chloralformamid durch Reaktion mit Säurechloriden zunächst das N-(1,2,2,2-Tetrachlorethyl)-formimidchlorid hergestellt (DE-OS 24 58 827):

$$CCl_3-\overset{OH}{CH}-NH-CHO \xrightarrow{\text{Säurechlorid}} CCl_3-\overset{Cl}{CH}-N=CHCl,$$

das dann in einem zweiten Reaktionsschritt (DE-OS 24 58 825) mit Schwefel gemäß

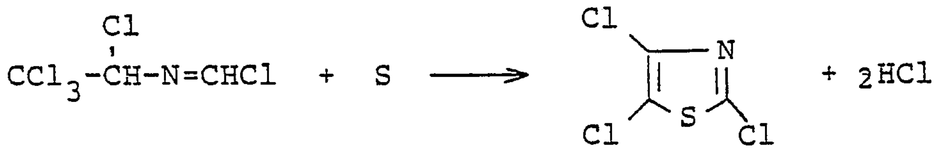

zum Trichlorthiazol umgesetzt wird.

Beim zweiten Verfahren wird aus dem Chloralformamid durch Reaktion mit Säurechloriden und Chlor zunächst das 1,2,2,2-Tetrachlorethyl-isocyaniddichlorid hergestellt (DE-OS 24 58 826):

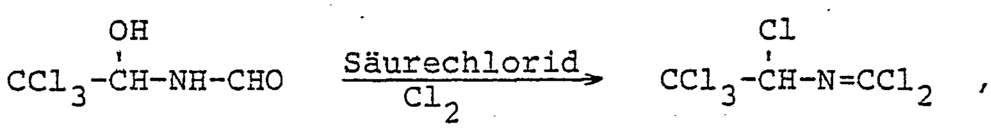


das dann in einem zweiten Reaktionsschritt (DE-OS 24 58 824) mit Schwefel gemäß

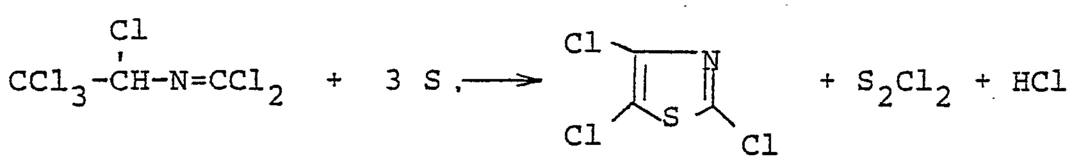


zum Trichlorthiazol umgesetzt wird.

Beide Verfahren haben alle Nachteile der Zweistufigkeit, wie zeitraubende Zwischendestillationen der jeweils ersten Stufen sowie insgesamt mehr Aufwand an Apparaturen etc. Darüber hinaus liefert das zweite Verfahren bei etwa verdoppelter Reaktionszeit über beide Stufen (Zeit für Zwischenisolierung nicht miteingerechnet) eine (um ca. 9 %) niedrigere Ausbeute und produziert pro kg Trichlorthiazol ca. 0,7 kg unerwünschtes Schwefelchlorid. Das erste Verfahren hat außer der Zweistufigkeit den Nachteil, daß das

im ersten Reaktionsschritt herzustellende N-(1,2,2,2-Tetrachlorethyl)-formimidchlorid ein hochreaktives Zwischenprodukt darstellt, das u.a. wegen seiner leichten Hydrolysierbarkeit nur unter besonderen Vorsichtsmaßnahmen zu handhaben ist. Außerdem ist beim zweiten Reaktionsschritt in der Praxis ein 80 %iger Überschuß an Schwefel nötig.

Es wurde nun überraschend gefunden, daß man Trichlorthiazol der Formel

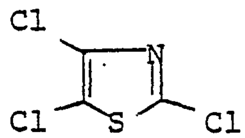

(I)

erhält, wenn man Chloralformamid der Formel

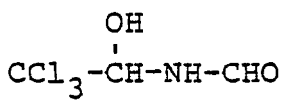

(II)

pro Mol mit mindestens zwei Mol Thionylchlorid unter Zusatz von 0,01 bis 10 Volumen-% eines N-alkylsubstituierten aliphatischen Säureamids, bezogen auf Thionylchlorid, und mit mindestens einem Mol Schwefel bei Temperaturen beginnend bei Raumtemperatur bis zu einem Bereich von 150-250°C umsetzt.

Der bevorzugte Bereich der Endtemperatur liegt bei 190-210°C.

Das Verfahren der Erfindung sei anhand folgender Reaktionsgleichung näher erläutert:

$CCl_3-\overset{OH}{CH}-NH-CHO + 2\ SOCl_2 + S \longrightarrow$ 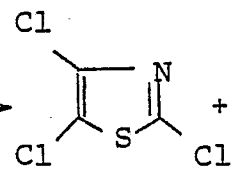 $+ 2\ SO_2 + 4\ HCl$

(II) (I)

Bei der Durchführung des erfindungsgemäßen Verfahrens setzt man auf 1 Mol der Ausgangsverbindung (II) mindestens 1 Mol Schwefel ein. Zur Erzielung eines vollständigen Umsatzes ist es jedoch zweckmäßig, Schwefel im Überschuß, zum Beispiel in einem bis zu 50 %igen Überschuß, einzusetzen. Bevorzugt wird die ca. 1,2-fache stöchiometrisch erforderliche Menge Schwefel zur Reaktion eingesetzt.

Weiterhin werden auf 1 Mol Ausgangsverbindung (II) mindestens 2 Mol Thionylchlorid eingesetzt; zweckmäßigerweise läßt man jedoch das Thionylchlorid gleichzeitig als Lösungs- bzw. Verdünnungsmittel fungieren, so daß bevorzugt das etwa 2- bis 3-fache der stöchiometrisch erforderlichen Menge an Thionylchlorid, d.h. etwa 4 bis 6 Mol Thionylchlorid pro Mol Chloralformamid, Anwendung findet. In Gew.- bzw. Volumenteilen ausgedrückt, bedeutet dies, daß pro Gewichtsteil Chloralformamid vorzugsweise etwa 2 Volumenteile Thionylchlorid zur Anwendung gelangen.

Das erfindungsgemäße Verfahren wird außerdem unter Zusatz eines N-alkylsubstituierten aliphatischen Säureamids durchgeführt. Im allgemeinen setzt man 0,01 bis 10 Volumen-%, vorzugsweise 0,5 bis 1,5 Volumen-% dieses Hilfsstoffes, bezogen auf Thionylchlorid, ein.

Als Beispiele für geeignete N-alkylsubstituierte aliphatische Säureamide seien genannt: Dimethylformamid, Methylformamid, Dimethylacetamid, N-Methylpyrrolidon, Tetramethylharnstoff. - Bevorzugt wird Dimethylformamid eingesetzt.

Das als Ausgangsprodukt verwendete Chloralformamid (II) ist bekannt (vgl. Ber. dtsch. Chem. Ges. 45 (1912), S. 945).

Zur Durchführung des Verfahrens der Erfindung vereinigt man bei Raumtemperatur die Ausgangs- bzw. Hilfsstoffe Thionylchlorid, N-alkylsubstituiertes aliphatisches Säureamid, Schwefel und Chloralformamid und rührt die Reaktionsmischung zunächst ohne Erwärmen so lange, bis die anfangs starke Gasentwicklung etwas nachgelassen hat. Dann wird in dem Maße, daß die Gasentwicklung nicht zu heftig wird, bis auf Rückflußtemperatur und bis zum Ende der Gasentwicklung erhitzt. Anschließend wird das überschüssige Thionylchlorid abdestilliert und steht für Folgeumsetzungen zur Verfügung. Die Reaktionstemperatur wird nun wiederum in dem Maße, daß die Gasentwicklung nicht zu heftig wird, bis auf 190 bis 210°C gesteigert und dort bis zum Ende der Reaktion (d.h. keine Gasentwicklung mehr, keine Bildung und Abdestillieren von Schwefelchloriden, die durch eine Nebenreaktion gebildet werden, mehr) gehalten. Anschließend wird in üblicher Weise destillativ aufgearbeitet.

Das Trichlorthiazol der Formel (I) ist bekannt (vgl. DE-OS 22 13 865) und zeigt insektizide Eigenschaften. Es kann außerdem als Zwischenprodukt zur Herstellung von bestimmten herbiziden Wirkstoffen verwendet werden (vgl. DE-OS 29 14 003 und EP-A 18 497).

Beispiel

In einem 4-Liter-Dreihalskolben, der mit Rührer, Thermometer und einer etwa 1 m langen Silbermantelkolonne mit Kolonnenkopf versehen ist, wird eine Mischung aus 2000 ml (entsprechend 3276 g ≙ 27,53 Mol) Thionylchlorid, 20 ml Dimethylformamid und 200 g (6,25 Mol) Schwefel mit 1000 g (5,195 Mol) Chloralformamid bei Raumtemperatur versetzt. Nach dem Nachlassen der starken Gasentwicklung (ca. 40 Minuten) wird langsam innerhalb von 2 Stunden bis auf Rückflußtemperatur (85 bis 89°C) erwärmt und dort solange (2-3 Stunden) gehalten, bis die Gasentwicklung praktisch beendet ist (5 bis 6 Stunden ab Reaktionsbeginn). Anschließend wird bei nun geöffnetem Kolonnenkopf das überschüssige Thionylchlorid im Verlauf von etwa 1,5 Stunden abdestilliert, wobei bei einer Heizbadtemperatur von 130 bis 180°C die Sumpftemperatur bis auf etwa 150°C ansteigt. Durch weitere allmähliche Erhöhung der Heizbadtemperatur steigert man die Sumpftemperatur bis auf 200 bis 205°C und hält dort solange (ca. 12 Stunden), bis keine Gasentwicklung mehr zu beobachten ist und keine Schwefelchloride mehr abdestillieren (Abnahme von Destillat bis zu einem Übergang von maximal 130°C). Gesamter Zeitbedarf ab Reaktionsbeginn ca. 23 Stunden.

Nach dem Abkühlen wird die Kolonne durch eine Destillationsbrücke ersetzt; im Wasserstrahlvakuum werden

alle bis zu einer Heizbadtemperatur von 220°C abdestillierbaren Anteile abdestilliert. Man erhält 776 g eines Destillats, das nach der gaschromatographischen Analyse zu 93,9 % aus Trichlorthiazol (I) besteht. Das entspricht einer Ausbeute von 728,7 g (≙ 74,4 % der Theorie).

Durch Feindestillation an einer 1 m-Kolonne wird das Trichlorthiazol bei 76-78°C/16 mbar in einer gaschromatographisch bestimmten Reinheit von 99-100 % erhalten. Es stimmt in allen seinen Eigenschaften mit dem gemäß DE-OS 22 13 865 hergestellten Trichlorthiazol überein.

Selbstverständlich kann nach Beendigung der Reaktion das Trichlorthiazol auch direkt über die 1 m-Silbermantelkolonne fraktioniert destilliert werden.

Patentansprüche

1. Verfahren zur Herstellung von Trichlorthiazol der Formel

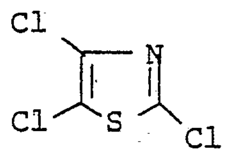

(I)

dadurch gekennzeichnet, daß man Chloralformamid der Formel

$$CCl_3-CH(OH)-NH-CHO \qquad (II)$$

pro Mol mit mindestens zwei Mol Thionylchlorid unter Zusatz von 0,01 bis 10 Volumen-% eines N-alkylsubstituierten aliphatischen Säureamids, bezogen auf Thionylchlorid, und mit mindestens einem Mol Schwefel bei Temperaturen beginnend bei Raumtemperatur bis zu einem Bereich von 150-250°C umsetzt.

2. Verfahren nach Anspruch 1, dadurch gekennzeichnet, daß man bei einer Endtemperatur im Bereich von 190-210°C arbeitet.

3. Verfahren nach Anspruch 1, dadurch gekennzeichnet, daß man pro Mol Chloralformamid die doppelte bis dreifache stöchiometrisch erforderliche Menge (d.h. 4-6 Mol) Thionylchlorid einsetzt.

4. Verfahren nach Anspruch 1, dadurch gekennzeichnet, daß man pro Mol Chloralformamid ca. 1,2 Mol Schwefel einsetzt.

5. Verfahren nach Anspruch 1, dadurch gekennzeichnet, daß man ca. 0,5-1,5 Volumen-% eines N-alkylsubstituierten aliphatischen Säureamids, bezogen auf Thionylchlorid, einsetzt.

6. Verfahren nach Anspruch 1, dadurch gekennzeichnet, daß man als N-substituiertes aliphatisches Säureamid Dimethylformamid einsetzt.

0053765

Nummer der Anmeldung

Europäisches Patentamt

# EUROPÄISCHER RECHERCHENBERICHT

EP 81 10 9944

| EINSCHLÄGIGE DOKUMENTE | | | KLASSIFIKATION DER ANMELDUNG (Int. Cl.³) |
|---|---|---|---|
| Kategorie | Kennzeichnung des Dokuments mit Angabe, soweit erforderlich, der maßgeblichen Teile | betrifft Anspruch | |
| DA | DE - A - 2 458 824 (BAYER AG)<br>* Seite 1-3 *<br>-- | 1 | C 07 D 277/32 |
| DA | DE - A - 2 458 825 (BAYER AG)<br>* Seite 1-3 *<br>-- | 1 | |
| DA | DE - A - 2 458 826 (BAYER AG)<br>* Seite 11 *<br>-- | 1 | RECHERCHIERTE SACHGEBIETE (Int. Cl.³)<br>C 07 D 277/00 |
| DA | DE - A - 2 458 827 (BAYER AG)<br>* Seite 1-5 *<br>---- | 1 | |

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze
E: älteres Patentdokument, das jedoch erst am oder nach dem Anmeldedatum veröffentlicht worden ist
D: in der Anmeldung angeführtes Dokument
L: aus andern Gründen angeführtes Dokument

&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 01-03-1982 | BRIGHENTI |

EPA form 1503.1 06.78